# EUROPEAN PATENT SPECIFICATION

(11) **EP 2 763 615 B1**
(45) Date of publication and mention of the grant of the patent: **13.11.2019**
(21) Application number: 12778510.3
(22) Date of filing: 24.09.2012
(51) Int. Cl.: A61B 18/04, A61B 17/29, A61B 17/32, A61B 18/14, A61B 17/00, A61B 18/12, A61B 34/30

(54) **LAPAROSCOPIC INSTRUMENT WITH ATTACHABLE ENERGY END EFFECTOR**
LAPAROSKOPISCHES INSTRUMENT MIT EINEM DARAN BEFESTIGBAREN ENERGIEENDEFFEKTOR
INSTRUMENT DE LAPAROSCOPIE DOTÉ D'UN EFFECTEUR TERMINAL ÉLECTRIQUE AMOVIBLE

(30) Priority: 30.09.2011 US 201113249790
(43) Date of publication of application: 13.08.2014
(73) Proprietor: Ethicon Endo-Surgery, Inc., Cincinnati, OH 45242 (US)
(72) Inventor: HOUSER, Kevin L., Springboro, Ohio 45066 (US); NOBIS, Rudolph H., Mason, Ohio 45040 (US)
(74) Representative: Carpmaels & Ransford LLP
(86) International application number: PCT/US2012/056900
(87) International publication number: WO 2013/048963

(56) References cited:
- WO-A1-2011/044353
- US-A1- 2011 087 265

## Description

### BACKGROUND

The present invention relates in general to surgical devices and more particularly to minimally invasive surgery.

Surgical procedures are often used to treat and cure a wide range of diseases, conditions, and injuries. Surgery often requires access to internal tissue through open surgical procedures or endoscopic surgical procedures. The term "endoscopic" refers to all types of minimally invasive surgical procedures including laparoscopic, arthroscopic, natural orifice intraluminal, and natural orifice trans luminal procedures. Endoscopic surgery has numerous advantages compared to traditional open surgical procedures, including reduced trauma, faster recovery, reduced risk of infection, and reduced scarring. Endoscopic surgery is often performed with an insufflatory fluid present within the body cavity, such as carbon dioxide or saline, to provide adequate space to perform the intended surgical procedures. The insufflated cavity is generally under pressure and is sometimes referred to as being in a state of pneumoperitoneum. Surgical access devices are often used to facilitate surgical manipulation of internal tissue while maintaining pneumoperitoneum. For example, trocars are often used to provide a port through which endoscopic surgical instruments are passed. Trocars generally have an instrument seal, which prevents the insufflatory fluid from escaping while an instrument is positioned in the trocar.

WO 2011/044353 and US 2011/0087265 describe and illustrate a surgical device having an elongate shaft defining a longitudinal axis, the shaft comprising a distal end and a proximal end, and an arm comprising a mating feature, the arm being axially slideable relative to the elongate shaft and being medially deflectable. An elongate pin is positioned medially relative to the arm, the elongate pin being axially slideable relative to the arm between a locked position preventing medial deflection of the arm and an unlocked position allowing medial deflection of the arm. A surgical end effector is selectively attachable in vivo and detachable in vivo to the mating feature of the arm. A lateral notch is present on the distal end of the elongate shaft and a mating feature on the surgical end effector, the mating feature comprising a ring dimensioned to mate with the arm lateral notch. A handle is connected to the proximal end of the elongate shaft, and has a trigger that controls the axial movement of the arm and an actuator that controls the axial movement of the elongate pin. The end effector has cooperating jaws that move between open and closed positions in response to axial motion of the arm. While surgical access devices are known, no one has previously made or used the surgical devices and methods in accordance with the present invention.

### SUMMARY

The invention is defined in the appended claims. Aspects, embodiments and examples disclosed herein which do not fall within the scope of the appended claims do not form part of the invention, and are merely provided for illustrative purposes.

A surgical device according to the invention comprises an elongate shaft defining a longitudinal axis. The shaft comprises a distal end and a proximal end. An arm medially deflectable and comprises a mating feature. An elongate pin is positioned medially relative the arm. The elongate pin is axially slideable relative the arm between a locked position preventing medial deflection of the arm and an unlocked position allowing medial deflection of the arm. An energy based surgical end effector is selectively attachable and detachable to the mating feature of the arm. The end effector includes a torque arm to engage the elongate shaft.

The energy based end effector may be an ultrasonic end effector. The proximal end of the elongate pin may be attached to an ultrasonic transducer wherein ultrasonic energy is transmitted to the elongate pin from the transducer. The elongate pin may have a length equal to an integral number of half wavelengths at the driven frequency of the transducer. The ultrasonic end effector may include an ultrasonic blade. The proximal end of the ultrasonic blade may include a tapered section wherein the elongate pin includes a tapered section. The tapered section of the ultrasonic blade and the elongate pin may be designed to attach the elongate pin to the ultrasonic blade such that ultrasonic energy from the elongate pin is transferred to the ultrasonic blade. The device may include a second arm identical to the first arm and positioned opposing the first arm on the opposite side of the longitudinal axis of the device wherein the space between the two arms defines a slot. The elongate pin may include a feature that rides in the slot between the two arms and wherein at least one of the arms includes a feature that extends at least partially into the slot. The feature on the arm may interact with the feature on the elongate pin to prevent the elongate pin from moving distally until a predetermined force is applied to the pin.

In another embodiment, the energy based end effector is a RF end effector. The elongate pin may be electrically connected to a RF surgical generator where the pin forms one side of the RF circuit. The RF end effector may comprises two jaws, each jaw comprising an electrode electrically connected to the elongate pin, the electrodes being adapted to contact tissue grasped between the jaws. The arm may be electrically connected to a RF surgical generator, the arm forming one side of the bipolar RF circuit. The RF end effector may comprises two jaws, each jaw comprising an electrode electrically connected to the arm, the electrodes being adapted to contact tissue grasped between the jaws.

In yet another embodiment, the surgical device may comprise a housing attached to the proximal end of the shaft. The housing may be a robotic interface having features that connect the housing to a robotic actuation arm, wherein the features permit the robotic actuation arm to move the elongate pin to lock and unlock end effectors from the surgical device. An energy based surgical generator may be located inside the housing. A battery may be located in the housing for powering the surgical generator.

### BRIEF DESCRIPTION OF DRAWINGS

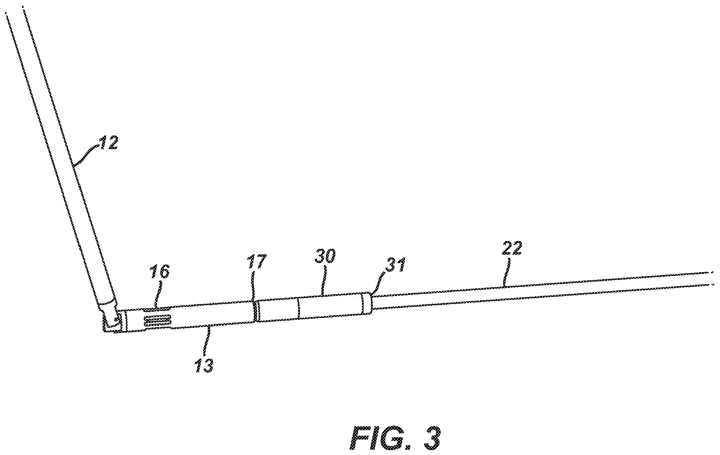

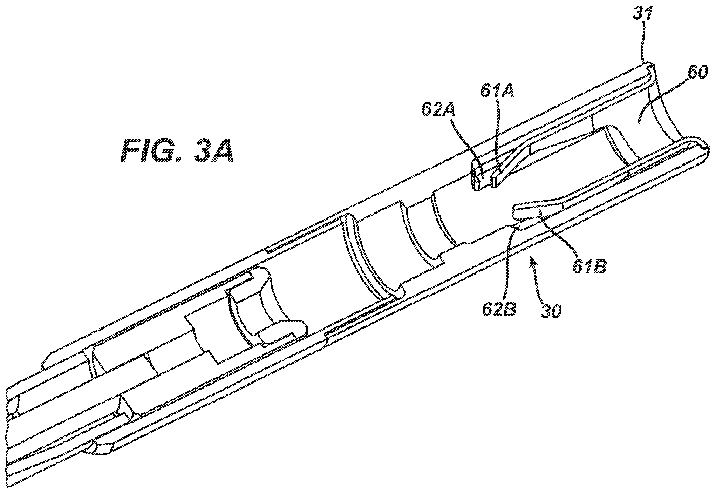

While the specification concludes with claims which particularly point out and distinctly claim the invention, it is believed the invention will be better understood from the following description taken in conjunction with the accompanying drawings illustrating some non-limiting examples of the invention. Unless otherwise indicated, the figures are not necessarily drawn to scale, but rather to illustrate the principles of the invention. The torque arm of the present invention is depicted in figures 3A - 3E. The remaining figures are included to provide background and context.
Fig. 1 depicts surgical procedure with an instrument and loader holding an end effector;

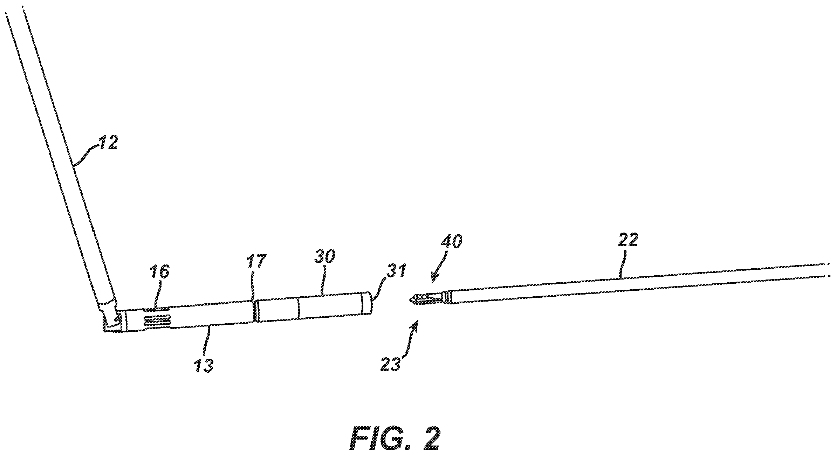

Fig. 2 depicts a close-up view of the distal ends of the instrument and loader in Fig. 1;
Fig. 3 depicts an instrument being inserted into an end effector;
Fig. 3A depicts an isometric cross-sectional view of an end effector;
Fig. 3B depicts an isometric cross-sectional view of an instrument partially inserted into an end effector;
Fig. 3C depicts an end effector with torque arms provided in the lateral surface of the end effector;
Fig. 3D depicts a close up of the end effector of Fig. 3C;
Fig. 3E depicts a cross section of the Fig. 3D end effector with an instrument inserted in the end effector;
Fig. 4 depicts an instrument attached to an end effector being withdrawn from a loader;
Fig. 4A depicts a loader with removable distal end;
Fig. 5 depicts an isometric close-up view of the distal end of an instrument in a locked position;
Fig. 6 depicts an isometric close-up view of the distal end of an instrument in an unlocked position;
Fig. 7 depicts an isometric cross-sectional view of the distal end of an instrument attached to an end effector;
Fig. 7A depicts an isometric cross-sectional view of the distal end of an instrument attached to an end effector with the pin advanced distally;
Fig. 8 depicts an isometric cross-sectional view of the distal end of an instrument attached to an end effector in an unlocked and uncoupled configuration;
Fig. 9 depicts an instrument handle;
Fig. 10 depicts a bi-polar jawed end effector;
Fig. 11 depicts a cutting shears end effector;
Fig. 12 depicts a Maryland dissector end effector;
Fig. 13 depicts an ultrasonic shears end effector;
Fig. 14 depicts an isometric close-up view of the distal end of an ultrasonic instrument in a locked position;
Fig. 15 depicts an isometric close-up view of the distal end of a bipolar RF based instrument with a tissue cutting knife in a locked position;
Fig. 16 depicts an isometric close-up view of the distal end of a bipolar RF based instrument with a tissue cutting knife in a locked position;
Fig. 17 depicts a side sectional view of an ultrasonic end effector;
Fig. 18 depicts an isometric view of a bipolar RF end effector with a knife;
Fig. 19 depicts an ultrasonic surgical device connected to a generator;
Fig. 20 depicts a variation to connect the elongate pin;
Fig. 21 depicts a bipolar end effector;
Fig. 22 depicts an instrument handle connected to a generator;
Fig. 23 depicts a shaft with robotic housing; and
Fig. 24 depicts a detailed view of the robotic housing.

### DETAILED DESCRIPTION

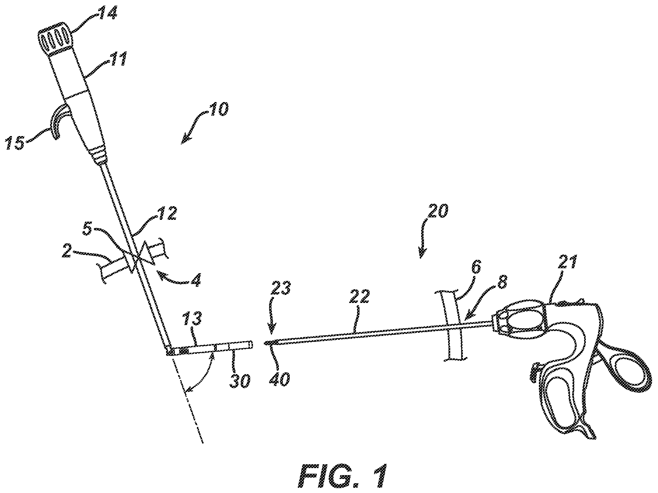

As shown in Fig. 1, instrument (20) comprises an elongate shaft (22) passing through an incision (8) of a tissue wall (6). A loader (10) comprises an elongate shaft (12) passing through an incision (4) of a tissue wall (2). The surgical end effector (30) is selectively attachable in vivo and detachable in vivo to the attachment mechanism (40) located at the distal end (23) of the instrument (20). In this example, the end effector is a jawed tissue grasper, but a variety of other end effectors could be also be used. The end effector (30) may be loaded ex vivo into the distal end (13) of the shaft (12), and then introduced into the surgical field through the incision (4). The loader (10) holds the end effector (30) during the in vivo attachment to and in vivo detachment from the instrument (20). The loader (10) and instrument (20) each includes ex vivo handles (11, 21) attached to the proximal ends of the shafts (12, 22) that enable surgeons to use the devices

The tissue wall (2,6) anatomies will vary based on the surgical procedure, but some nonlimiting examples include percutaneous incisions into the abdomen, thorax, or pelvis. The incisions (4, 8) may be created with a cutting or puncturing instrument, and will typically be spaced from one another. The tissue walls (2, 6) may be the same or different anatomies. For instance, tissue walls (2, 6) may both be the abdominal wall. In another example, tissue wall (2) could be an organ (e.g., stomach, colon, esophagus, etc.) accessed through a natural orifice, while the incision (8) in tissue wall (6) could be percutaneous. In yet another example, incision (4) may provide access to the abdomen, while the incision (8) may provide access to the pelvis. If pneumoperitoneum is desired, the incisions may include instrument seals, such as those commonly found in trocars. In this example, the instrument seal (5) is schematically shown in incision (4) with the loader (10) passing through the seal (5), while the shaft (22) seals directly with the tissue wall (6) by virtue of the resilience of the tissue without the aid of a sealing device.

The loader shaft (12) in this device is rigid and straight, but the shaft (12) could be curved or flexible, which would be beneficial for natural orifice trans luminal introduction of the distal end (13) to the surgical field. The loader (10) may include an articulating distal end (13) controlled by the knob (14). The distal end (13) will typically be introduced and removed through the incision (4) in-line with the shaft (12), and then articulated in vivo to facilitate alignment between the end effector (30) and the shaft (22). The arm (15) is rigidly connected the handle (11) to facilitate grasping of the handle and rotational orientation of the articulated distal end (13) about the shaft (12) axis. In this device, the distal end (13) of the loader (10) comprises a tube opening at the distal tip (17). The tube is dimensioned to receive the end effector (32). The tube (30) includes an engagement feature (16) for holding the end effector (32). While the engagement feature (16) may vary, in this device a plurality of leaf springs provide an interference fit with the end effector (30) to frictionally hold the end effector in the tube. In this device, when the end effector (30) is loaded in the tube, the distal end (32) is positioned in the tube and the proximal end (31) extends from the tube opening (17). This arrangement prevents the jaws of the end effector from opening. After the distal end (23) of the instrument (20) is attached to the proximal end (31) of the end effector (30), the end effector (3) can be pulled from the distal end (13) of the loader (10).

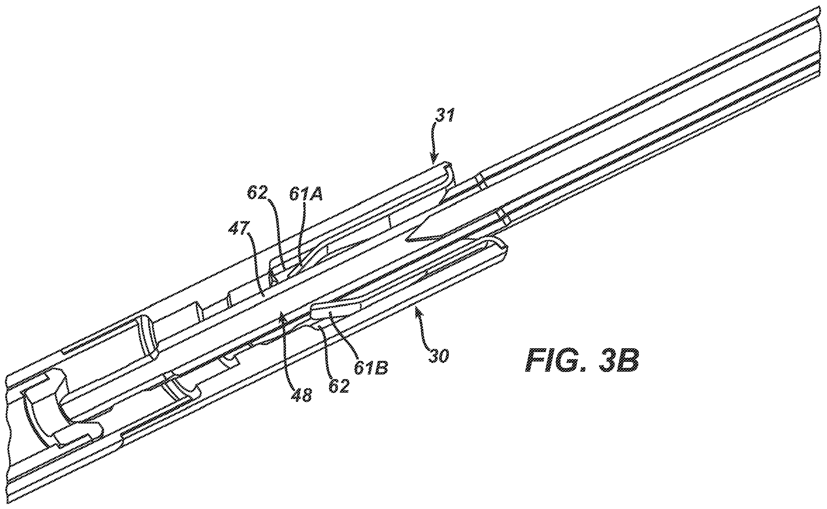

Fig. 3A depicts an example of an end effector provided with a torque key (60). The torque key, in one expression, is fixedly attached to proximal end (31) of end effector (30). Torque key (60) is provided with torque arms (61A, 61B). Torque arms (61) may be provided with a medial angular bend. End effector (30) may also be provided with torque arm recesses (62A, 62B) that permit the torque arms (61) to laterally deflect creating a variable inner diameter of end effector (30). Fig. 3B depicts the instrument shaft (22) partially inserted into end effector (30). In this depiction, torque arms (61) are aligned with flat surfaces on the shaft arms (47) and protrude medially into an opening (48) between shaft arms (47). When shaft (22) is inserted into end effector (30) and the torque arms (61) are not aligned with opening (48), they will remain deflected medially in recess (62) until the shaft (22) is rotated to align torque arms (62) with opening (48). When aligned with the opening (48), torque arms (61) permit transfer of rotational force from the shaft to the end effector.

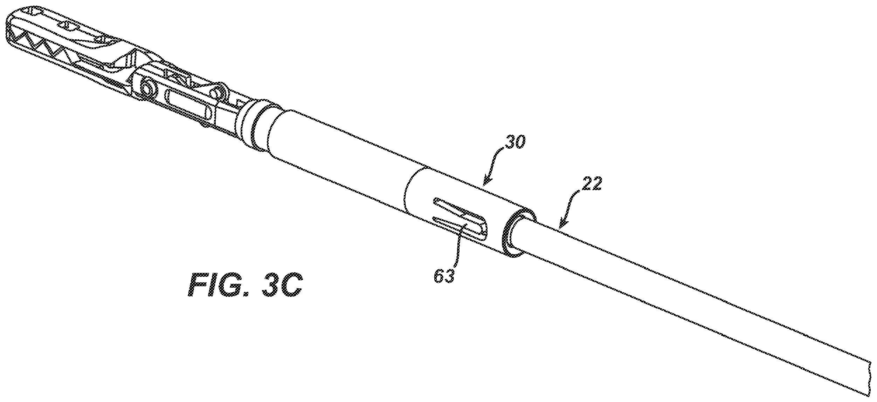

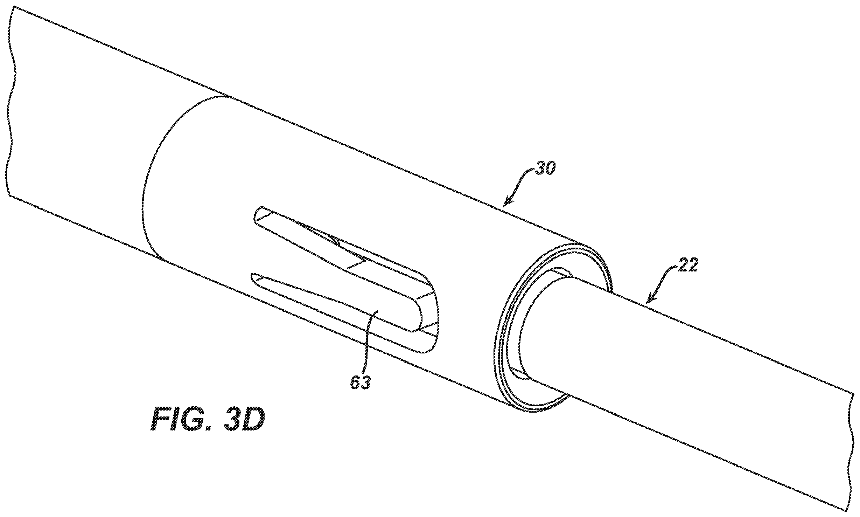

Figs. 3C and 3D depict another expression of the end effector (30). The proximal end of the end effector (30) is provided with flexible torque arm (63) formed from the lateral surface of end effector (30). When shaft (22) is inserted into end effector (30), torque arm (63) may deflect laterally where the opening (48) is not aligned with torque arm (63). To facilitate engagement with shaft (22) torque arm (63) may be provided with a chamfered surface. Upon rotation of the shaft (22), the torque arm will align with opening (48). When aligned with the opening (48), torque arm (63) permits transfer of rotational force from the shaft (22) to the end effector (30).

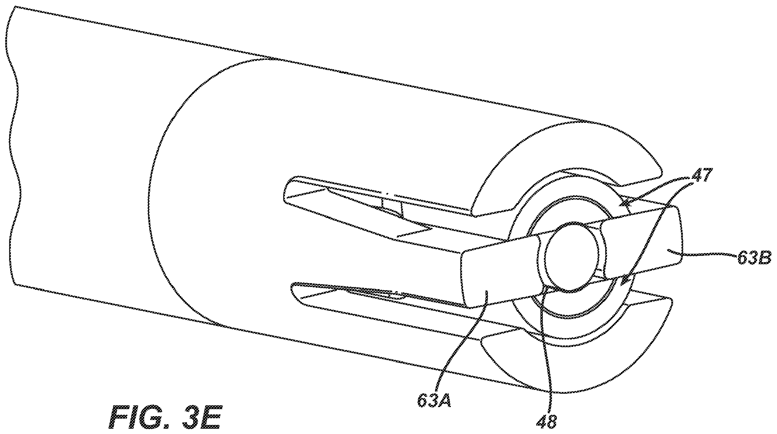

Fig. 3E depicts a cross sectional view of a shaft (22) inserted into end effector (30). In this expression, end effector (30) is provided with two torque arms (63A, 63B). Torque arms (63) are aligned to opening (48) defined by shaft arms (47) creating an interference fit.

In another expression of the surgical instrument, the torque arms (63) may be provided with recessed inner portions that mate with projections on the lateral surface of the shaft (not shown). The shaft projections may be flexible to facilitate entry of the shaft into the end effector. In yet another expression, the end effector may be provided with recesses (not shown) located on the medial surface of the end effector that mate with the projections on the lateral surface of the shaft.

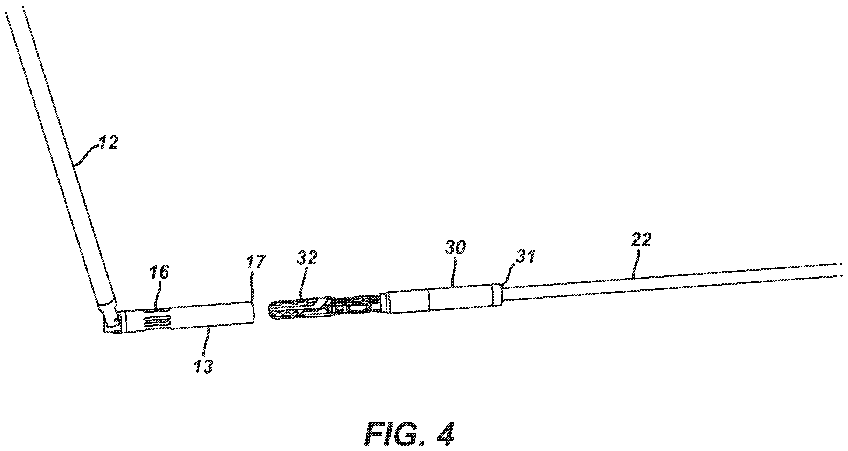

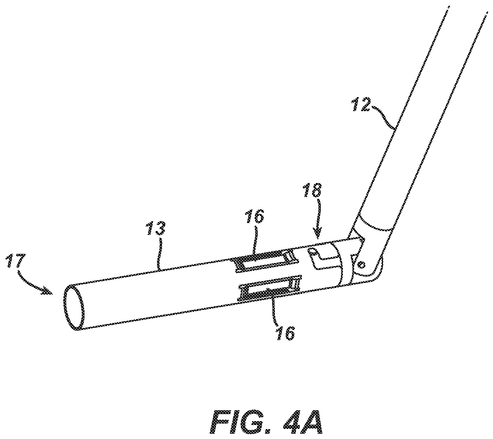

Fig. 4 depicts an end effector (30) attached to an instrument (20) being withdrawn from a loader (13). Fig. 4A depicts an alternative loader (10) where the distal end (13) is selectively attachable and detachable to the shaft (12). As shown in this example, this feature is enabled with a bayonet connection (18), but other connections are also contemplated including snap connections, threaded connections, and the like. One advantage of this alternative device is that different distal end (13) configurations may be used to hold end effectors that may not be accommodated by a single sized tube.

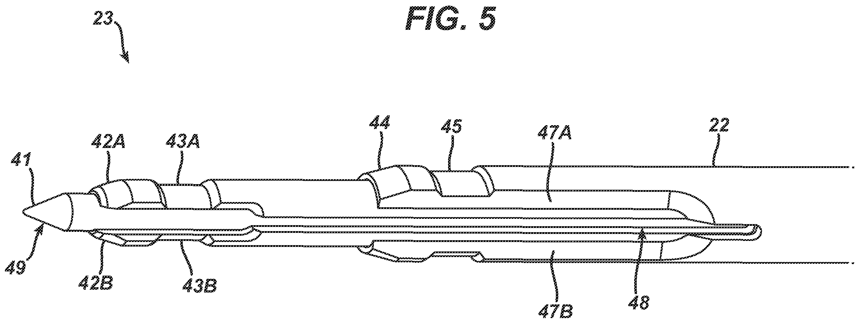

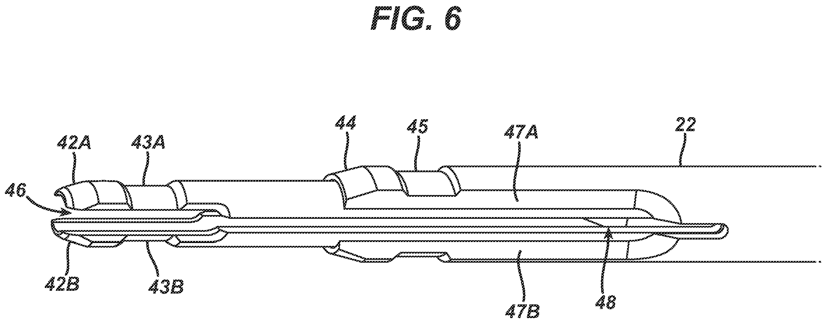

Figs. 5 and 6 depict a detailed view of an attachment mechanism (40) located at the distal end (23) of the shaft (22). The attachment mechanism (40) comprises a mating feature on the shaft (22), which in this device is a circumferential groove (45) positioned on the lateral surface of shaft arms (47A, 47B). Shaft arms (47A, 47B) may be resiliently flexible into opening (48). The attachment mechanism (40) also comprises second arms (42A, 42B) projecting distally from the distal end (44) of the shaft (22). The second arms may be axially slideable relative the shaft (22) and are resiliently deflectable medially into the gap (46). The second arms each comprise a mating feature, which in this device comprises a stepped lateral notch (43A, 43B). An elongate pin (41) is positioned medially relative the second arms (42) and shaft arms (47) and is axially slideable relative the second arms (42) and shaft arms (47) between a locked position preventing medial deflection of the arms (42 and 47) (an example of which is shown in Fig. 5) and an unlocked position allowing medial deflection of the arms (an example of which is shown in Fig. 6). The pin (41) and second arms (42) may each slide independently relative the shaft (22) and shaft arms (47). FIG 6 shows the pin (41) fully retracted inside shaft (22) allowing medial deflection of shaft arms (47).

As shown in the device of Fig. 5, the elongate pin (41) may include a pointed obtrurator tip. In this configuration the distal end (23) may be used to puncture through the tissue wall (6). The distal ends of the second arms (42) and distal end (44) of the shaft arms (47A, 47B) include tapered surfaces to facilitate passing through the incision (8).

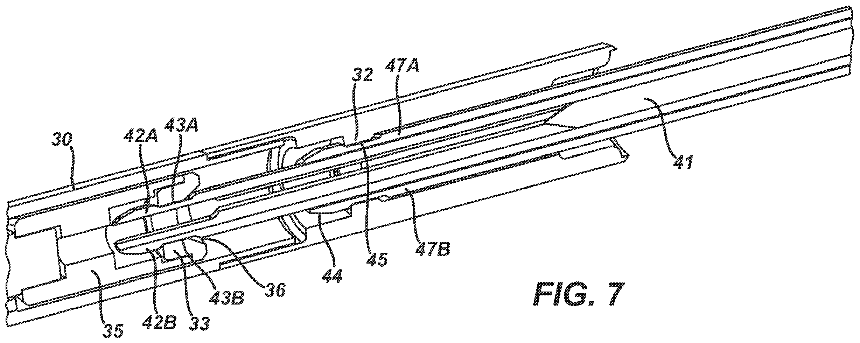

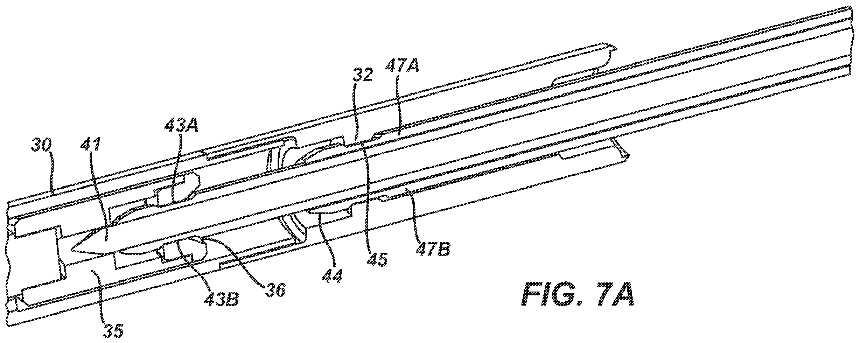

Fig. 7 shows the attachment mechanism (40) attached to the end effector (30). The groove (45) of the shaft arms (47) mates the rib (32) of the end effector (30) preventing relative axial motion. The lateral grooves (43A, 43B) of the second arms (42) mate the ring (33) of the end effector (30) preventing relative axial motion. The rib (32) is rigidly connected to the outer housing (37) of the end effector (30), and the ring (33) is rigidly connected to the jaw actuator (34) via the coupling (35). When the elongate pin (41) is fully advanced, medial deflection of the second arms (42) and the shaft arms (47) is inhibited (see Fig. 7 A). Accordingly, axial movement of the arms (42) relative the shaft (22) will cause axial movement of the jaw actuator (34) relative the housing (37), thereby causing the jaws to open and close.

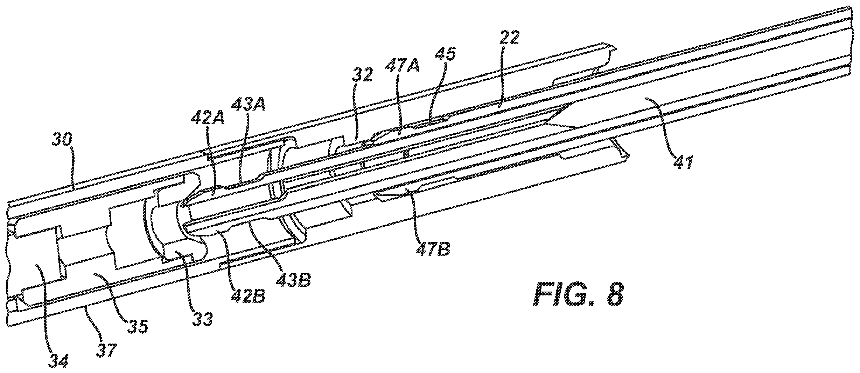

After completing the surgical procedure, the end effector (30) may be detached from the shaft (22). If previously removed, the loader (10) may be reintroduced through the seal (5) into the surgical field. The distal end (32) of the end effector (30) is seated into the distal end (13) of the loader (10), and the pin (41) moved to its unlocked position. The second arms (42) are then proximally withdrawn from the ring (33), deflecting medially as the chamfered portions of the second arms (42) slide over the ring (33) medial surfaces. Accordingly, the device will be in the configuration depicted in Fig. 8. Proximally retracting the arms (47) will cause the shaft arms (47) to deflect medially into the opening (48) as the chamfered portions of shaft arms (47) slide over the rib's (32) medial surfaces which simultaneously cause the second arms (42) to deflect medially into the gap (46) facilitating easier separation of the end effector (30) from the shaft (22). The proximal advancement of the shaft (22) continues until the rib (32) unseats from the groove (45). This unseating may be facilitated by the jaws of the end effector (30) being held in a closed position by the tube in the loader distal end (13). The distal end (23) may then be withdrawn from the end effector (30) thus detaching the end effector (30) from the instrument (20). The end effector will be held in the loader (10) by virtue of the engagement feature (16). Removal of the loader (10) from the surgical field will remove the end effector (30). A different end effector may then be attached to the instrument (20), or the instrument (20) may be withdrawn from the surgical field.

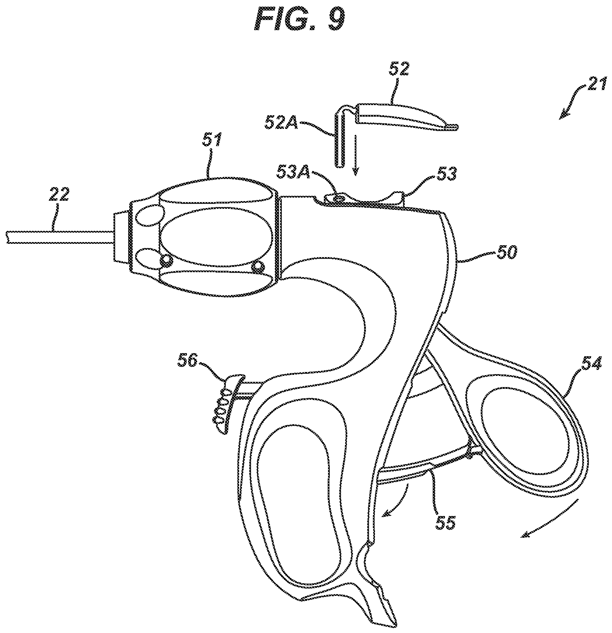

Fig. 9 shows and example of the handle (21) for the instrument (20). The handle (21) includes a base (50). A knob (51) rotates the attachment mechanism (40) about the axis of the shaft (22), which will also rotate an attached end effector (30). The trigger (54) pivots relative the base (50) causing axial movement of the second arms (42) and the pin (41) relative the shaft (22). Operation of the trigger (54) will operate the jaws on an attached end effector (30). The latch (55) pivots relative the base (50) between a locked position (as shown in figure) to prevent operation of the trigger (54) and an unlocked position recessed in the base (50). During seating with the end effector (30), the latch (55) may be locked to maintain the same relative axial spacing of the corresponding the mating features (43, 45) as the mating features (33, 32), resulting in resulting in a single "snap" feedback. The trigger lock (56) can lock/unlock the trigger in/from its depressed position. An actuator (53), which in this device is a slider, controls axial movement of the pin (41) relative the second arms (42). The distal most position of the actuator (53) relative the base (as shown in the figure) places the pin (41) in its locked position, and the proximal most position places the pin (41) in its unlocked position. The pin lock (52) includes a pin (52A) which went inserted into the hole (53A) maintains the pin (41) and second arms (42) in the extended and locked positions as shown in Fig. 5.

The following describes one method for attaching the end effector (30) to the shaft (22). The distal end (23) is introduced in into the proximal end (31) of the end effector (30) with the pin (41) in the unlocked position. The shaft (22) deflects the torque arms (61) laterally into recesses (62) when the torque arms are not aligned with the opening (48). In another expression, torque arm (63) deflects laterally upon shaft (22) insertion into the end effector (30). When the torque arm (61, 63) are aligned with the opening (48), they do not deflect and rest adjacent to opening (48) on the lateral surfaces of shaft arms (47) permitting rotation of the end effector. As the arms (42) are advanced axially into the end effector (30), the chamfered lead (36) of the ring (33) medially deflects the arms (42) until the ring (33) is seated into the lateral notches (43). Simultaneously the shaft arms (47) advance axially into the end effector (30), and the tapered end (44) aligns the rib (32) to seat into the groove (45). In both cases, the surgeon may feel a tactile "click" indicating proper engagement. Once fully seated in the end effector (30), the pin (41) may be slid to the locked position thereby attaching the end effector (30) to the instrument (20). Once attached, the surgeon may pull the end effector from the loader (10), and the loader (10) may then be removed from the surgical field. When the end effector (30) is attached to the shaft (22) and the torque arm (61, 63) are not aligned with the opening (48), the surgeon may grip tissue or another instrument and rotate the knob (51) until the torque arms (61) seat in the opening (48). The surgeon may then manipulate tissue with the end effector (30) as needed for the surgical procedure.

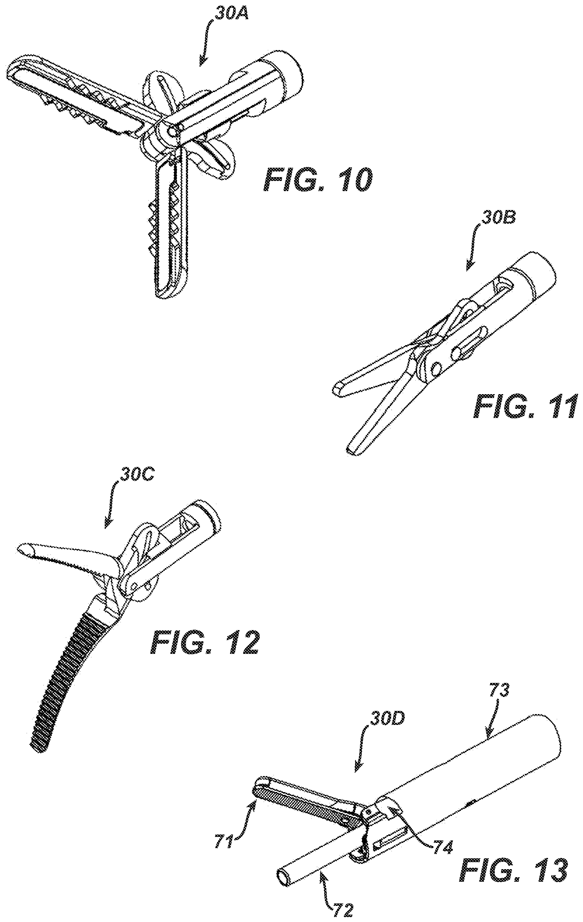

Figs. 10-13 illustrate some non-limiting examples of alternative end effectors (30A-D) that may attached to the distal end (23) of the instrument (20). In addition to the loader (10) and instrument (20), all or a portion of the end effectors (30, 30A, 30B, 30C, 30D) may be bundled as part of a kit so the surgeon may interchange the attached end effector as needed for a surgical procedure. All the end effectors examples shown here have cooperating jaws; however, nonjawed end effectors could also be employed such as hook knives, snares, and the like. In the case of end effectors that require energy, appropriate energy transmission mechanisms known in the art should be added to the handle (21) and shaft (22). For instance, appropriate electrical connections can be added for the bi-polar forceps end effector (30A). Similarly, an ultrasonic transducer and waveguide can be added for the ultrasonic shears end effector (30D).

The following describes one method for using the devices during a laparoscopic surgical procedure. An instrument (20) is obtained and passed through incision (8). The incision (8) may be a percutaneous incision formed at least partially by a puncture formed with the obtruator on the pin (41) in the configuration shown in Fig. 5. The pin lock (52) and latch (55) may be secured to the actuator (53) and trigger (54), respectively. After the puncture, the pin lock (52) may be removed, and the actuator (53) may be fully retracted proximally.

A loader (10) and end effector (30) are obtained. The end effector (30) may be selected from a plurality of end effectors provided in a kit. The end effector (30) is loading ex vivo into the distal end (13) of the loader (10). The distal end (13) of the loader (10) with the loaded end effector (30) is passed through incision (4). The second incision (4) may also be percutaneous incision spaced from the first incision (8), and may include passing the distal end (13) with the loaded end effector (30) through a trocar. The distal end (13) may be articulated to facilitate orientation between the proximal end (31) of the end effector (30) and the attachment mechanism (40). The actuator (53) is slid proximally to move the pin (41) to its unlocked position. The distal end (23) of the instrument (20) is advanced into the proximal end (31) of the end effector (30) until the respective mating features of the instrument (20) and end effector (30) are engaged. The actuator (53) may then be slid distally thus advancing the pin (41) to its locked position. The end effector (30) has now been attached in vivo to the instrument (20). The end effector (30) may then be pulled from the loader (10) and the latch (55) disengaged from the trigger (54). Tissue is then manipulated by actuating the trigger (54) of the handle (21) to operate the jaws of the end effector (30).

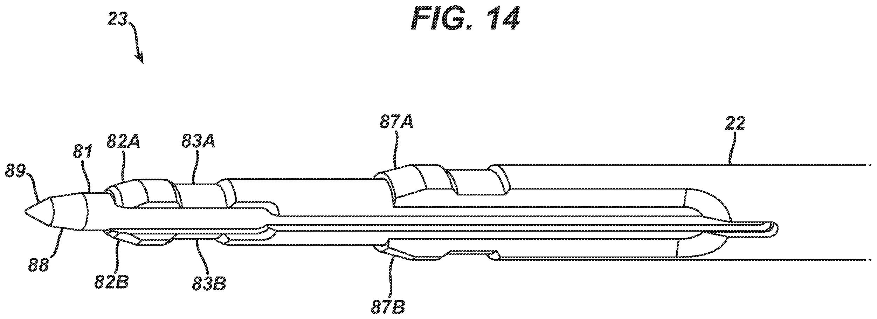

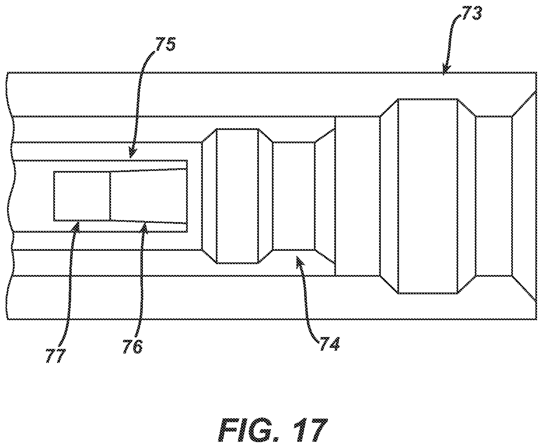

Fig. 14 shows an alternative attachment mechanism (80) located at the distal end (23) of the shaft (22). Attachment mechanism (80) is designed to allow for the connection of an ultrasonic end effector (30D) as shown in Fig. 13. An acoustically stable coupling is provided between the distal end (23) of the shaft (22) and the ultrasonic end effector (30D). Similar to the device shown in Figs. 5 and 6, an elongate pin (81) slides independently of shaft arms (87A, 87B) and second arms (82A, 82B). Shaft arms (87A, 87B) engage the outer tube (73) on ultrasonic end effector (30D). Second arms (82A, 82B) engage inner tube (74) on ultrasonic end effector (30D). Once inner tube (74) and outer tube (73) are engaged, elongate pin (81) is pushed forward to engage the proximal portion (75) of ultrasonic blade (72) shown in Fig. 17. Proximal portion (75) of ultrasonic blade (72) includes a tapered surface (76) which engages with tapered portion (88) of elongate pin (81). One example of an appropriate acoustic connection is described in US patent 6,561,983.

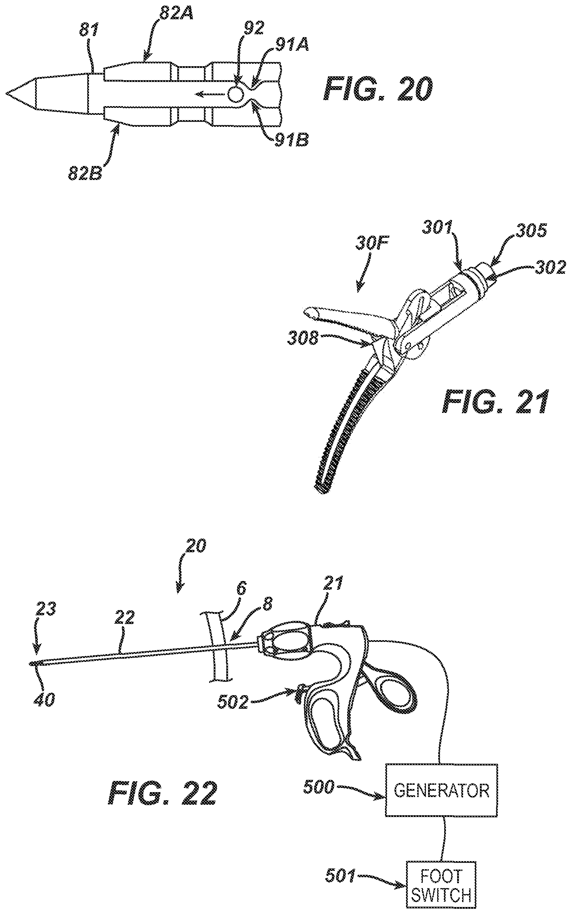

The connection between the ultrasonic blade (72) and the elongate pin (81) may have a relatively high force applied to them in order to facilitate a proper connection. For example, the method to create this force is shown in Fig. 20 wherein second arms (82) include proximal ramps (91A, 91B). A small pin (92) protrudes from the elongate pin (81) and as the user advances the elongate pin (81) its motion is resisted by proximal ramps (91) until sufficient force is built up by the user to overcome the resistance. This then causes the elongate pin (81) to spring forward with sufficient force to create a satisfactory acoustic joint between the elongate pin (81) and the ultrasonic blade (72). Other methods for creating this force, including springs or over-center mechanisms, are also contemplated.

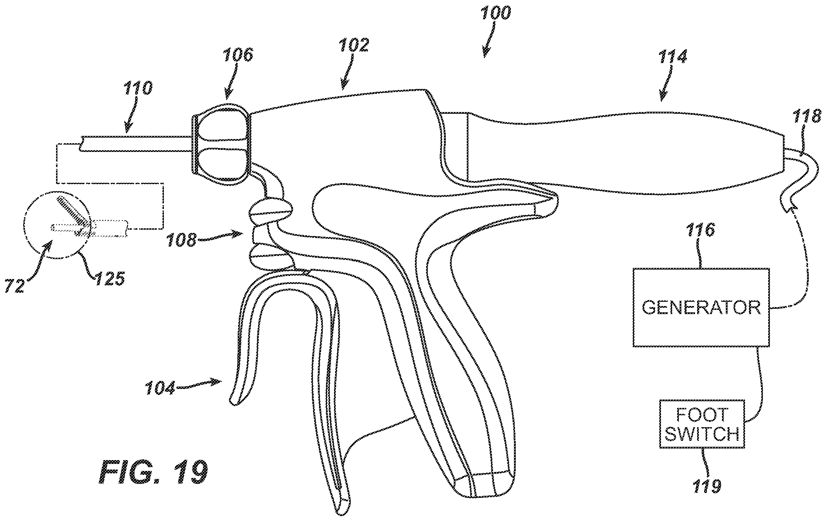

The ultrasonic blade (72) is preferably an even number of acoustic half wave segments, the half wave of the blade (72) being a function of the natural frequency of the blade (72) and the material used in the blade (72). The ultrasonic blade (72) shown vibrates in a longitudinal mode, however other modes of vibration such as torsion and transverse vibration may also be used. Other fractions of a full wave may also be used. The ultrasonic blade (72) may be fixed to the outer tube (73) at an acoustic nodal point, the acoustic nodal point defined as a point on the blade where the primary mode of vibration is at minimum amplitude. The ultrasonic blade (72) may be fixed to the outer tube (73) using a pin, a snap fit, or any other fixation method known in the art. With ultrasonic end effector (30D) locked onto shaft (22) movement of trigger (104) shown in Fig. 19 causes associated movement of second arms (82) and inner tube (74) which in turn causes clamp arm (71) to close down onto ultrasonic blade (72). Various methods of connecting the trigger (104) to the clamp arm (71) are known, including those shown in US patent application serial number 11/246,826. In the device shown, the elongate pin (81) is connected at its proximal end (not shown) to ultrasonic transducer (114) by screw threads, press fit, or any other mechanical connection means.

Elongate pin (81) has a length equivalent to an integral number of acoustic half wavelengths. To puncture the abdomen and insert the shaft (22) the user can apply pressure to the instrument (100), using the sharp tip section (89) of the elongate pin (81) to penetrate the tissue. Alternatively, the distal tip (89) may be blunt instead of sharp and rely the ultrasonic energy to piece tissue. By pressing either hand activation switch (108) or foot switch (119), the ultrasonic generator (116) provides power to the ultrasonic transducer (114) which in turn causes ultrasonic motion of the elongate pin (81). This motion allows the user to push the sharp tip section (89) of elongate pin (81) through the tissue with reduced force and with improved hemostatic effects. Although the ultrasonic generator (116) is shown as a separate unit, other devices are contemplated wherein the ultrasonic generator (116) and power supply (not shown) are incorporated into the instrument (100). For instance, the instrument (100) can be powered by a battery (not shown).

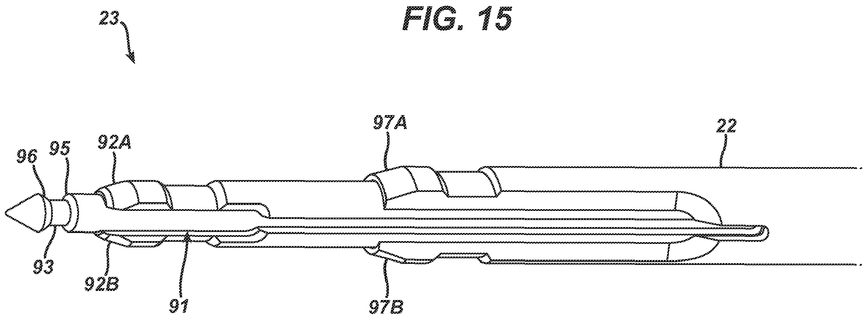

Fig. 15 shows an alternative attachment mechanism (90) located at the distal end (23) of the shaft (22). Attachment mechanism (90) is designed to allow for the connection of a bipolar end effector with a knife (30F) as shown in Fig. 21. An electrically stable coupling is provided between the distal end (23) of the shaft (22) and the bipolar end effector with a knife (30F). An elongate pin (91) slides independently of shaft arms (97A, 97B) and second arms (92A, 92B). Shaft arms (97A, 97B) engage the outer tube (301) on bipolar end effector with a knife (30F). Second arms (92A, 92B) engage jaw driver (302) on bipolar end effector with a knife (30F). Once inner tube (304) and outer tube (303) are engaged, elongate pin (91) is pushed forward to engage the proximal portion (305) of knife (308) shown in Fig. 21. Proximal portion (305) of knife (308) includes a snap fit engagement surface (306) which engages with recessed portion (93) of elongate pin (91). The attachment surface (95) proximal to recessed portion (93) is a step that allow the elongate pin (91) to provide force to advance the knife (308) when the elongate pin (91) is advanced. The attachment surface (96) distal to recessed portion (93) is ramped to allow for easier disassembly. In this device, elongate pin (91) provides the ground potential side of the RF circuit and second arms (92) provide the high potential side of the RF circuit.

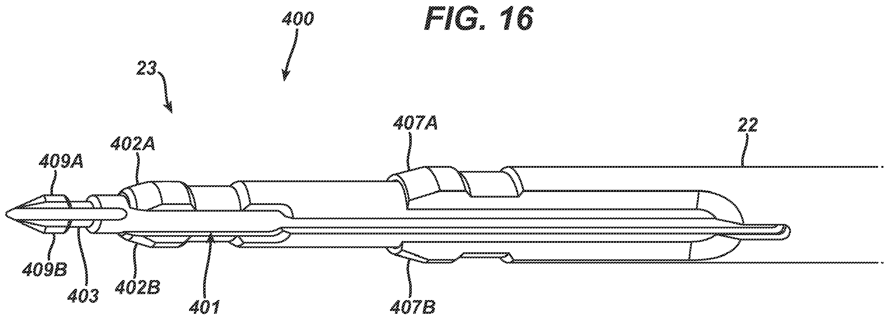

In an alternative device shown in Fig. 16, third arms (409A, 409B) are included. Proximal portion (305) of knife (308) engages into recess (403). Advancing elongate pin (401) locks proximal (305) of knife (308) onto attachment mechanism (400). This allows for devices wherein the force to retract the knife (308) is sufficiently high that it would cause the proximal end (305) of the knife (308) to disengage from the attachment mechanism (90) shown in Fig. 15. In this device, third arms (409) provide the ground potential side of the RF circuit and second arms (402) provide the high potential side of the RF circuit. In any of these devices the ground and high potential sides of the RF circuit can be switched without changing the intent of this design. Also, in any of these devices, elongate pins, arms, second arms or third arms can be attached to either the high side potential or the ground potential provided that each of these is electrically connected to the appropriate electrode in the jaws and knife of the device.

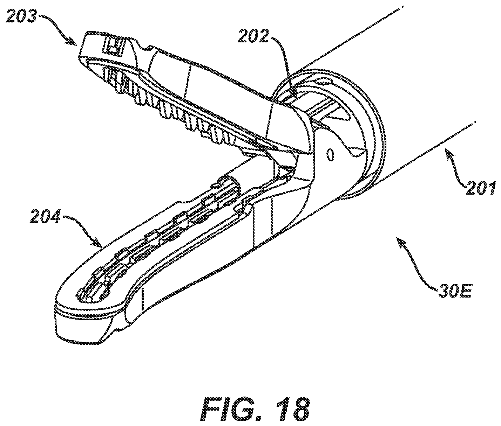

Fig. 18 depicts an alternative bipolar end effector with knife (30E) for use with attachment mechanism (40) shown in Fig. 5. Arms (47) engage with outer tube (201). Second arms (42) engage with knife (202). Advancement of knife (202) causes upper jaw (203) to close down onto lower jaw (204). Mechanisms of this type are shown in US Patent 7,381,209. Referencing Figs. 5, 14, 16, 21 and 22, to puncture the abdomen and insert the shaft (22) the user can apply pressure to the instrument (21), using the sharp tip section (49) of the elongate pin (41) to penetrate the tissue. Alternatively, the user can use the bipolar cutting properties of the instrument (20). By pressing either hand activation switch (502) or foot switch (501), the Bipolar RF generator (500) provides power to the end effector (30F). This energy allows the user to push the sharp tip section (49) of elongate pin (41) through the tissue with reduced force and with improved hemostatic effects. In an alternative, the distal tip (49) may be blunt instead of sharp, thus relying the RF energy to piece tissue. Although the ultrasonic generator (500) is shown as a separate unit, other devices are contemplated wherein the ultrasonic generator (500) and power supply (not shown) are incorporated into the instrument (20). For instance, the instrument (20) can be powered by a battery (not shown).

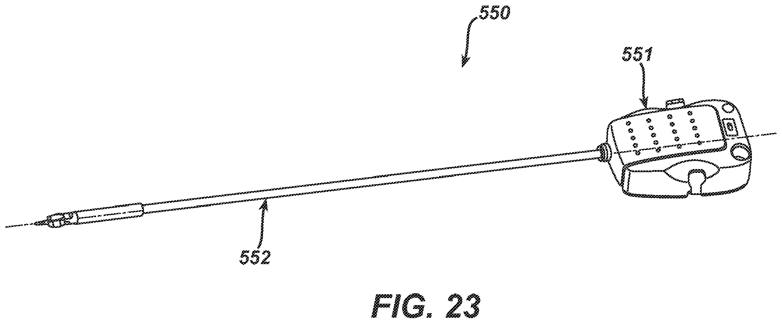

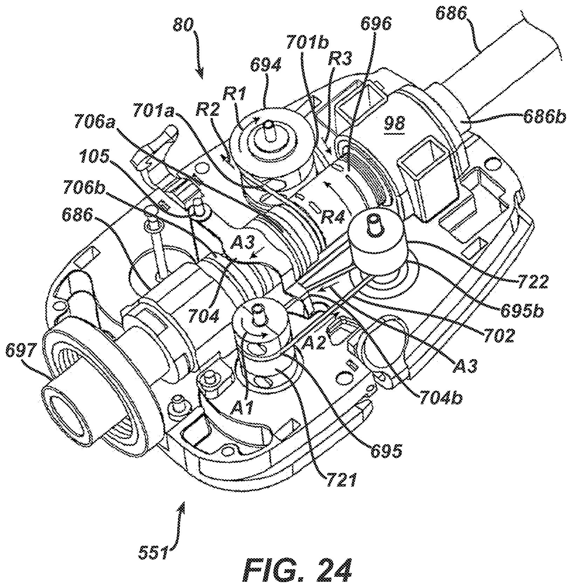

Figs. 23 and 24 depict a version of the instrument (550) that is adapted for use on a robotic surgical station like that shown in US patent 6,783,524. In this device, the handle (21 in Fig. 1) portion is replaced by a robotic interface mechanism (551). Internal to the robotic interface mechanism (551) are a series of drive gears (694, 721, 722)). Connection of the end effectors (30) is accomplished in the same means as with the hand held instruments however, in this device, the axial motion of the elongate pin (41, Fig. 5) is created by drive gear (721). Motion of the second arms (42, Fig. 5) is driven by drive gear (722) and rotation of the instrument relative to the longitudinal axis is driven by drive gear (694). Motion of the elongate pin (41, Fig. 5) when done to lock the attachment mechanism may or may not be initiated by the hand controls (not shown) on the surgical robot. Alternatively, foot switches, hand switches, motion algorithms that read specific hand motions as activation indications or any other actuation means known in the art may be used. Also more complex versions of the attachment mechanism (400, Fig. 16) may also make use of additional gears (not shown) within the robotic interface mechanism (551). It is also contemplated that the electrical inputs from the surgical robot could be used to power an energy based generator located inside the robotic interface mechanism.

Without limitation, the following describe some of the benefits and advantages of the foregoing devices and methods over the prior art. The end effector (30) may have a much larger diameter than the shaft (22); accordingly, the incision (8) can be smaller compared to more traditional laparoscopic instruments resulting in less pain and scarring, and quicker recovery. This also facilitates a small diameter shaft (22) (even less than 3mm), thus potentially eliminating a trocar in the incision (8). The attachment mechanism (40) provides quick end effector (30) exchanges with the instrument (20), thus decreasing surgical time. The loader (10) also facilitates quick end effector (30) exchanges. A kit of multiple end effectors may reduce instrument costs by consolidating a single shaft (22) and handle (21) for all instruments. Many other benefits will be apparent to those skilled in the art.

Adaptations of the devices described herein can be accomplished by appropriate modifications by one of ordinary skill in the art without departing from the scope of the present invention. Several of such potential modifications have been mentioned, and others will be apparent to those skilled in the art. For instance, the specific materials, dimensions, and the scale of drawings will be understood to be non-limiting examples. Accordingly, the scope of the present invention should be considered in terms of the following claims and is understood not to be limited to the details of structure, or materials shown and described in the specification and drawings.

Having shown and described various embodiments and examples of the present invention, further adaptations of the methods and devices described herein can be accomplished by appropriate modifications by one of ordinary skill in the art without departing from the scope of the present invention. Several of such potential modifications have been mentioned, and others will be apparent to those skilled in the art. For instance, the specific materials, dimensions, and the scale of drawings will be understood to be non-limiting examples. Accordingly, the scope of the present invention should be considered in terms of the following claims and is understood not to be limited to the details of structure, materials, or acts shown and described in the specification and drawings.

## Claims

1. A surgical device (20), comprising:
a) an elongate shaft (22) defining a longitudinal axis, the shaft comprising a distal end (23) and a proximal end;
b) an arm (42A, 42B; 82A, 82B; 92A, 92B; 402A, 402B) comprising a mating feature, the arm being medially deflectable;
c) an elongate pin (41, 81, 91, 401) positioned medially relative the arm, the elongate pin being axially slideable relative the arm between a locked position preventing medial deflection of the arm and an unlocked position allowing medial deflection of the arm; and
d) an energy based surgical end effector (30, 30A, 30B, 30C 30D, 30E, 30F) selectively attachable and detachable to the mating feature of the arm,
**characterized by** the end effector having a torque arm (61, 63) to engage the elongate shaft (22).

2. The surgical device (20) of claim 1, wherein the energy based end effector (30D) is an ultrasonic end effector (30D).

3. The surgical device (20) of claim 1, wherein the energy based end effector is a RF end effector (30E, 30F).

4. The surgical device (20) of claim 2, wherein the proximal end of the elongate pin is attached to an ultrasonic transducer (114) and wherein ultrasonic energy is transmitted to the elongate pin from the transducer.

5. The surgical device (20) of claim 4, wherein the elongate pin has a length equal to an integral number of half wavelengths at the driven frequency of the transducer.

6. The surgical device (20) of claim 2, wherein the ultrasonic end effector includes an ultrasonic blade (72), the proximal end (75) of the ultrasonic blade including a tapered section (76), and wherein the elongate pin (81) includes a tapered section (88), the tapered section (76) of the ultrasonic blade (72) and the elongate pin (81) being designed to attach the elongate pin (81) to the ultrasonic blade (72) such that ultrasonic energy from the elongate pin (81) is transferred to the ultrasonic blade (72).

7. The surgical device (20) of claim\ 6, wherein the device includes a second arm (82A, 82B) identical to the first arm and positioned opposing the first arm on the opposite side of the longitudinal axis of the device and wherein the space between the two arms defines a slot.

8. The surgical device (20) of claim 7, wherein at the elongate pin (81) includes a feature (92) that rides in the slot between the two arms (82A, 82B) and wherein at least one of the arms includes a feature (91A, 91B) that extends at least partially into the slot, the feature (91A, 91B) on the arm (82A, 82B) interacting with the feature (92) on the elongate pin (81) to prevent the elongate pin (81) from moving distally until a predetermined force is applied to the pin.

9. The surgical device (20) of claim 3, wherein the elongate pin (91) is electrically connected to a RF surgical generator (500), the pin forming one side of the RF circuit.

10. The surgical device (20) of claim 9, wherein the RF end effector (30F) comprises two jaws, each jaw comprising an electrode electrically connected to the elongate pin (91), the electrodes being adapted to contact tissue grasped between the jaws.

11. The surgical device (20) of claim 3, wherein the arm (92A, 92B) is electrically connected to a RF surgical generator (500), the arm (92A, 92B) forming one side of the bipolar RF circuit.

12. The surgical device (20) of claim 11, wherein the RF end effector (30F) comprises two jaws, each jaw comprising an electrode electrically connected to the arm, the electrodes being adapted to contact tissue grasped between the jaws.

13. The surgical device of claim 1, further comprising a housing (21, 80, 551) attached to the proximal end of the shaft (22).

14. The surgical device (20) of claim 13, wherein the housing is a robotic interface (551) having features that connect the housing to a robotic actuation arm and wherein the features permit the robotic actuation arm to move the elongate pin to lock and unlock end effectors from the surgical device.

## Patentansprüche

1. Chirurgische Vorrichtung (20), umfassend:
a) einen langgestreckten Schaft (22), der eine Längsachse definiert, wobei der Schaft ein distales Ende (23) und ein proximales Ende umfasst;
b) einen Arm (42A, 42B; 82A, 82B; 92A, 92B; 402A, 402B), der ein Passmerkmal umfasst, wobei der Arm medial ablenkbar ist;
c) einen langgestreckten Stift (41, 81, 91, 401), der relativ zu dem Arm medial positioniert ist, wobei der langgestreckte Stift axial relativ zu dem Arm zwischen einer arretierten Position, die eine mediale Ablenkung des Arms verhindert, und einer entarretierten Position gleitfähig ist, die mediale Ablenkung des Arms gestattet; und
d) einen auf Energie basierten chirurgischen Endeffektor (30, 30A, 30B, 30C 30D, 30E, 30F), der an dem Passmerkmal des Arms selektiv befestigbar und abnehmbar ist,
**dadurch gekennzeichnet, dass** der Endeffektor einen Drehmomentarm (61, 63) zum Eingriff mit dem langgestreckten Schaft (22) aufweist.

2. Chirurgische Vorrichtung (20) nach Anspruch 1, wobei der auf Energie basierte Endeffektor (30D) ein Ultraschallendeffektor (30D) ist.

3. Chirurgische Vorrichtung (20) nach Anspruch 1, wobei der auf Energie basierte Endeffektor ein HF-Effektor (30E, 30F) ist.

4. Chirurgische Vorrichtung (20) nach Anspruch 2, wobei das proximale Ende des langgestreckten Stifts an einem Ultraschallwandler (114) befestigt ist, und wobei Ultraschallenergie aus dem Wandler an den langgestreckten Stift übertragen wird.

5. Chirurgische Vorrichtung (20) nach Anspruch 4, wobei der langgestreckte Stift eine Länge gleich einer ganzzahligen Anzahl von halben Wellenlängen bei der angetriebenen Frequenz des Wandlers aufweist.

6. Chirurgische Vorrichtung (20) nach Anspruch 2, wobei der Ultraschallendeffektor eine Ultraschallklinge (72) einschließt, das proximale Ende (75) der Ultraschallklinge ein sich verjüngendes Segment (76) einschließt, und wobei der langgestreckte Stift (81) ein sich verjüngendes Segment (88) einschließt, wobei das sich verjüngende Segment (76) der Ultraschallklinge (72) und der langgestreckte Stift (81) so konzipiert sind, dass der langgestreckte Stift (81) so an der Ultraschallklinge (72) befestigt wird, dass Ultraschallenergie aus dem langgestreckten Stift (81) auf die Ultraschallklinge (72) übertragen wird.

7. Chirurgische Vorrichtung (20) nach Anspruch 6, wobei die Vorrichtung einen zweiten Arm (82A, 82B) einschließt, der mit dem ersten Arm identisch ist und an der entgegengesetzten Seite der Längsachse der Vorrichtung gegenüber von dem ersten Arm positioniert ist, und wobei der Raum zwischen den beiden Armen einen Schlitz definiert.

8. Chirurgische Vorrichtung (20) nach Anspruch 7, wobei der langgestreckte Stift (81) ein Merkmal (92) einschließt, das in dem Schlitz zwischen den beiden Armen (82A, 82B) sitzt, und wobei mindestens einer der Arme ein Merkmal (91A, 91B) einschließt, das sich mindestens teilweise in den Schlitz hinein erstreckt, wobei das Merkmal (91A, 91B) auf dem Arm (82A, 82B) mit dem Merkmal (92) auf dem langgestreckten Stift (81) interagiert, um den langgestreckten Stift (81) daran zu hindern, sich vor Einwirken einer vorbestimmten Kraft auf den Stift nach distal zu bewegen.

9. Chirurgische Vorrichtung (20) nach Anspruch 3, wobei der langgestreckte Stift (91) elektrisch mit einem chirurgischen HF-Generator (500) verbunden ist, wobei der Stift eine Seite der HF-Schaltung bildet.

10. Chirurgische Vorrichtung (20) nach Anspruch 9, wobei der HF-Endeffektor (30F) zwei Backen umfasst, wobei jede Backe eine Elektrode umfasst, die elektrisch mit dem langgestreckten Stift (91) verbunden ist, wobei die Elektroden vorgesehen sind, um in Kontakt mit Gewebe zu kommen, das zwischen den Backen gegriffen wurde.

11. Chirurgische Vorrichtung (20) nach Anspruch 3, wobei der Arm (92A, 92B) elektrisch mit einem chirurgischen HF-Generator (500) verbunden ist, wobei der Arm (92A, 92B) eine Seite der bipolaren HF-Schaltung bildet.

12. Chirurgische Vorrichtung (20) nach Anspruch 11, wobei der HF-Endeffektor (30F) zwei Backen umfasst, wobei jede Backe eine Elektrode umfasst, die elektrisch mit dem Arm verbunden ist, wobei die Elektroden vorgesehen sind, um in Kontakt mit Gewebe zu kommen, das zwischen den Backen gegriffen wurde.

13. Chirurgische Vorrichtung nach Anspruch 1, des Weiteren umfassend ein Gehäuse (21, 80, 551), das an dem proximalen Ende des Schafts (22) befestigt ist.

14. Chirurgische Vorrichtung (20) nach Anspruch 13, wobei das Gehäuse eine Roboterschnittstelle (551) mit Merkmalen ist, die das Gehäuse mit einem Roboterbetätigungsarm verbinden, und wobei die Merkmale gestatten, dass der Roboterbetätigungsarm den langgestreckten Stift bewegt, um Endeffektoren an der chirurgischen Vorrichtung zu arretieren und zu entarretieren.

## Revendications

1. Dispositif chirurgical (20), comprenant :
a) une tige allongée (22) définissant un axe longitudinal, la tige comprenant une extrémité distale (23) et une extrémité proximale ;
b) un bras (42A, 42B ; 82A, 82B ; 92A, 92B ; 402A, 402B) comprenant un élément d'accouplement, le bras étant déformable médialement ;
c) une broche allongée (41, 81, 91, 401) positionnée médialement par rapport au bras, la broche allongée pouvant être glissée axialement par rapport au bras entre une position verrouillée empêchant une déformation médiale du bras et une position déverrouillée permettant une déformation médiale du bras ; et
d) un effecteur terminal chirurgical à base d'énergie (30, 30A, 30B, 30C, 30D, 30E, 30F) attachable et détachable sélectivement de l'élément d'accouplement du bras,
**caractérisé en ce que** l'effecteur terminal a un bras de torsion (61, 63) pour s'enclencher avec la tige allongée (22).

2. Dispositif chirurgical (20) de la revendication 1, dans lequel l'effecteur terminal à base d'énergie (30D) est un effecteur terminal à ultrasons (30D).

3. Dispositif chirurgical (20) de la revendication 1, dans lequel l'effecteur terminal à base d'énergie est un effecteur terminal RF (30E, 30F).

4. Dispositif chirurgical (20) de la revendication 2, dans lequel l'extrémité proximale de la broche allongée est attachée à un transducteur ultrasonore (114) et dans lequel de l'énergie ultrasonore est transmise à la broche allongée depuis le transducteur.

5. Dispositif chirurgical (20) de la revendication 4, dans lequel la broche allongée a une longueur égale à un nombre entier de demi-longueurs d'onde à la fréquence imposée du transducteur.

6. Dispositif chirurgical (20) de la revendication 2, dans lequel l'effecteur terminal à ultrasons comporte une lame à ultrasons (72), l'extrémité proximale (75) de la lame à ultrasons comportant une section effilée (76), et dans lequel la broche allongée (81) comporte une section effilée (88), la section effilée (76) de la lame à ultrasons (72) et la broche allongée (81) étant conçues pour attacher la broche allongée (81) à la lame à ultrasons (72) de telle sorte que de l'énergie ultrasonore issue de la broche allongée (81) est transférée à la lame à ultrasons (72).

7. Dispositif chirurgical (20) de la revendication 6, le dispositif comportant un deuxième bras (82A, 82B) identique au premier bras et positionné en face du premier bras sur le côté opposé de l'axe longitudinal du dispositif, et l'espace entre les deux bras définissant une fente.

8. Dispositif chirurgical (20) de la revendication 7, dans lequel la broche allongée (81) comporte un élément (92) qui glisse dans la fente entre les deux bras (82A, 82B) et dans lequel au moins un des bras comporte un élément (91A, 91B) qui s'étend au moins partiellement dans la fente, l'élément (91A, 91B) sur le bras (82A, 82B) interagissant avec l'élément (92) sur la broche allongée (81) pour empêcher la broche allongée (81) de se déplacer distalement jusqu'à ce qu'une force prédéterminée soit appliquée à la broche.

9. Dispositif chirurgical (20) de la revendication 3, dans lequel la broche allongée (91) est reliée électriquement à un générateur chirurgical RF (500), la broche formant un côté du circuit RF.

10. Dispositif chirurgical (20) de la revendication 9, dans lequel l'effecteur terminal RF (30F) comprend deux mâchoires, chaque mâchoire comprenant une électrode reliée électriquement à la broche allongée (91), les électrodes étant adaptées pour toucher un tissu saisi entre les mâchoires.

11. Dispositif chirurgical (20) de la revendication 3, dans lequel le bras (92A, 92B) est relié électriquement à un générateur chirurgical RF (500), le bras (92A, 92B) formant un côté du circuit RF bipolaire.

12. Dispositif chirurgical (20) de la revendication 11, dans lequel l'effecteur terminal RF (30F) comprend deux mâchoires, chaque mâchoire comprenant une électrode reliée électriquement au bras, les électrodes étant adaptées pour toucher un tissu saisi entre les mâchoires.

13. Dispositif chirurgical de la revendication 1, comprenant en outre un boîtier (21, 80, 551) attaché à l'extrémité proximale de la tige (22).

14. Dispositif chirurgical (20) de la revendication 13, dans lequel le boîtier est une interface robotique (551) ayant des éléments qui relient le boîtier à un bras d'actionnement robotique, et dans lequel les éléments permettent au bras d'actionnement robotique de déplacer la broche allongée pour verrouiller et déverrouiller des effecteurs terminaux par rapport au dispositif chirurgical.
